# EUROPEAN PATENT APPLICATION

(11) **EP 2 540 281 A1**
(43) Date of publication of application: **02.01.2013**
(21) Application number: 11005339.4
(22) Date of filing: 30.06.2011
(51) Int. Cl.: A61K 9/107, A61K 9/16

(54) **Solid self-microemulsifying systems**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a self-microemulsifying system, preferably comprising active pharmaceutical ingredient, and a medicine prepared therefrom. This invention also relates to a process for preparing a solid self-microemulsifying system. In addition, it relates to a use of the self-microemulsifying system and a use of microcrystalline cellulose in admixture with the self-microemulsifying system. Specifically it describes a process for preparing a self-microemulsifying system comprising dispersing microcrystalline cellulose in a solvent, wherein the solvent further contains a viscosity enhancer, mixing the solvent with a self-microemulsifying system and spray drying the obtained mixture. In addition It provides the process comprising mixing a solid support and self-microemulsifying system to form a primary mixture and further granulating said primary mixture with a solution or suspension of a granulation binder. Both processes remarkably enhance processability of the solid self-microemulsifying system. Product and medicine obtainable by said processes are also provided.

## Description

### Field of the invention

The present invention relates to a self microemulsifying system, preferably comprising active pharmaceutical ingredient, and a medicine prepared therefrom. This invention also relates to a process for preparing a solid self-microemulsifying system. In addition, it relates to a use of the self-microemulsifying system and a use of microcrystalline cellulose in admixture with the self-microemulsifying system.

### Description of the background art

Self-microemulsifying systems (SMES) are liquid or semisolid systems consisting of a lipophilic phase with one or more surface active substance often termed as surfactant and one or more co-surfactant, and/or other excipients, which spontaneously form microemulsions upon the contact with the aqueous media. Compared to microemulsions, they do not contain water and thus exhibit better physical and microbiological stability. They share all the advantages of microemulsions over coarse emulsion. Incorporation of drugs in self-microemulsifying systems thus offers several advantages for their delivery, the main one being faster drug dissolution and absorption.

SMES present a hurdle when tried to be utilized as or formulated in a convenient dosage form, particularly due to problems associated with their physical characteristics such as stickiness, poor flowability and adhesion to laboratory/manufacturing equipment, which tremendously decreases yield of preparatory processes. As a consequence SMES are currently applied in liquid dosage form or as soft gelatin capsules, or they are transformed into solid state by adsorption/absorption of these systems onto solid carrier (also termed solid support). Solid state dosage systems have many advantages over liquid and semi-solid systems (including soft gelatin capsules), the main one being their better acceptance by the patients, their suitability and ease for the industrial manufacturing and better physico-chemical characteristics (e.g stability).

EP1961412A1 describes for possible alternatives to convert SMES into solid form. First possibility is to slowly add SMES to solid support during continuous vigorous mixing to obtain a free flowing powder. Second option is to stabilize individual emulsion phases with hydrophilic and hydrophobic solid supports. A water phase of a dispersion is adsorbed on a hydrophilic solid support by vigorous mixing and an oil phase of the dispersion is adsorbed on a hydrophobic solid support also by vigorous mixing. Afterwards, solid state water phase, solid state oil phase and emulsifier mixture are mixed to obtain a free flowing powder. Third possibility is to disperse SMES in a melted polyethylene glycol and rapidly s olidify it in an ice bath. The solidified composition is pulverized and sieved to form granulate. In the fourth case, the SMES is spray dried on a solid support, which is first dissolved or colloidally dispersed in a solvent, the obtained solution is mixed with the SMES and spray dried.

Thus obtained solid solutions still exhibit relatively low flowability and allow only mediocre yields in processes preparing them due to adhesion of solid SMES to laboratory/manufacturing equipment unless a substantial amount of solid support excipients is used to produce free flowing, readily compressible solid SMES. However, the amount of excipients added is limited by the final dosage form size and weight, which should not exceed a patient friendly size (e.g. 1000 mg). In addition, large quantities of excipients used often exceed the limits stated by the Code of Federal Regulation, which define allowable doses of excipients to be consumed daily.

The present invention provides improved processes for preparing solid self-microemulsifying system that overcome drawbacks of known processes by attaining increased yield and producing solid SMES with better handling properties in terms of improved flowability and less stickiness, wherein this is achieved by using completely acceptable amounts of excipients. By employing the processes of the present invention it is possible to transform in high yield SMES into solid form that can optionally be readily filled into capsules, compressed into tablets, or moulded into suppository alone or together with other pharmaceutically acceptable excipients. Solid SMES obtainable by said processes, and their use are also provided.

### Summary of the invention and its preferred embodiments

The aspects, advantages, features and preferred embodiments of the present invention summarized in the following independent and preferred items, respectively alone or in combination, are further provided by the invention:
(1) A process for preparing a solid self-microemulsifying system comprising:
   a) dispersing microcrystalline cellulose in a solvent,
   b) adding a viscosity enhancer in the solvent,
   c) mixing the solvent with a self-microemulsifying system to obtain a mixture, and
   d) spray drying the mixture.
(2) The process for preparing a solid self-microemulsifying system according to item 1, wherein the viscosity enhancer is selected from the group consisting of hydroxypropylmethyl cellulose, hydroxypropyl cellulose, hydroxyethyl cellulose, methylcellulose, carboxymethylcellulose, polyvinyl alcohols, polyvinylpirrolidones, copolyvidones, natural gums such as acacia, tragacanth, xanthan, carageenans, guar, pectin, sugars such as for example glucose, fructose, alginates, carbomers, gelatin, polyethylene glycols and colloidal silicon dioxide, or mixture thereof.
(3) The process for preparing a solid self-microemulsifying system according to item 1 or 2, wherein the viscosity enhancer is hydroxypropylmethyl cellulose.
(4) The process for preparing a solid self-microemulsifying system according to any one of items 1 to 3, wherein the solvent is selected from the group consisting of aqueous and organic solvents or a mixture thereof.
(5) The process for preparing a solid self-microemulsifying system according to any one of items 1 to 4, wherein the solvent is selected from the group consisting of water, ethanol, acetone, isopropanol, or a mixture thereof.
(6) The process for preparing a solid self-microemulsifying system according to any one of items 1 to 5, wherein the solvent is water or a mixture of water and ethanol, preferably the solvent is water.
(7) The process for preparing a solid self-microemulsifying system according to any one of items 1 to 6, wherein the microcrystalline cellulose in a solvent is in amount of 20 - 90 wt. % of the final solid self-microemulsifying system.
(8) The process for preparing a solid self-microemulsifying system according to any one of items 1 to 7, wherein the viscosity enhancer is in amount of 0.5 - 20 wt. % of the solvent.
(9) A process for preparing a solid self-microemulsifying system comprising:
   e) mixing a solid support and self-microemulsifying system to form a primary mixture, optionally in a presence of a granulating or spray drying liquid, and
   f) further granulating said primary mixture with a solution or suspension of a granulation binder, preferably of hydroxypropylmethyl cellulose, hydroxypropyl cellulose, hydroxyethyl cellulose, polyvinylpirrolidone, copolyvidone, methylcellulose, carboxymethylcellulose, gelatin, natural gum such as acacia, tragacanth, guar, pectin, or mixture thereof, more preferably of hydroxypropylmethyl cellulose, hydroxypropyl cellulose, hydroxyethyl cellulose, methylcellulose, ethylcellulose, carboxymethylcellulose, polyvinylpirrolidone, copolyvidone, or mixture thereof, particularly of hydroxypropylmethyl cellulose.
(10) The process for preparing a solid self-microemulsifying system according to item 9, wherein the solid support is lactose, microcrystalline cellulose, dibasic and tribasic calcium phosphate, silicon dioxide, kaolin, bentonite, hetorite, magnesium trisilicate, aluminium hydroxide, magnesium hydroxide, magnesium oxide, mannitol, talc, magnesium stearate, sodium chloride, potassium chloride, citric acid, starch, powdered cellulose, sorbitol, sucrose, calcium sulfate, sorbitol, xylitol, dicalcium hydrogen phosphate, or mixture thereof.
(11) The process for preparing a solid self-microemulsifying system according to items 9 or 10, wherein the solvent is selected from the group consisting of aqueous and organic solvents or a mixture thereof.
(12) The process for preparing a solid self-microemulsifying system according to any one of items 9 to 11, wherein the solvent is selected from the group consisting of water, ethanol, acetone, isopropanol, or a mixture thereof.
(13) The process for preparing a solid self-microemulsifying system according to any one of items 9 to 12, wherein the solvent is water or a mixture of water and ethanol, preferably the solvent is water.
(14) The process for preparing a solid self-microemulsifying system according to any one of items 9 to 13, wherein solid support is microcrystalline cellulose and the process further comprises steps of item 1.
(15) The process for preparing a solid self-microemulsifying system according to any one of previous items, the process further comprising adding an active pharmaceutical ingredient.
(16) The process for preparing a solid self-microemulsifying system according to item 15, wherein the active pharmaceutical ingredient is small molecule drug, nutritional supplement, peptide or protein, or a combination thereof.
(17) The process for preparing a solid self-microemulsifying system according to item 15 or 16, wherein the active pharmaceutical ingredient is selected from the group consisting of acetohexamide, albendazole, alendronate, amiodarone, aprepitant, atovaquone, bendroflumetiazid, benzthiazide, betamethasone, cyclosporine, danazol, diazepam, dutasteride, ezetimibe, felodipine, phenylbutazone, flufenaminic acid, fenofibrate, furosemide, glibenklamide, glutethimide, griseofulvin, hydroflumethiazide, hydrochlortiazide, hydrocortisone, ibuprofen, indomethacin, itraconazole, carbamazepine, ketoconazole, ketoprofen, clofibrate, chloramphenicol, chlorothiazide, clorpropamide, mebendazole, mefenaminic acid, megestrol, naproxen, nimodipin, nitrazepam, nitrendipine, nitrofurantoin, oxazepam, pioglitazone, prazosin, prednisolone, reserpine, simvastatine, sirolimus, spironolactone, telmisartan and troglitazone, or mixture thereof.
(18) The process for preparing a solid self-microemulsifying system according to any one of previous items, wherein the self-microemulsifying system comprises
   (i) a polyoxyethylene sorbitan fatty acid ester emulsifier;
   (ii) a co-emulsifier selected from glyceryl mono- or di- fatty acid esters of C6-C18 fatty acids; and
   (iii) an oil, selected from C6-C12 fatty acid triglycerides; wherein the polyoxyethylene sorbitan fatty acid ester emulsifier (i) and co-emulsifier (ii) are present in a ratio by weight of between 1:1 and 8:1 and the emulsifier and co-emulsifier, (i) + (ii) and oil (iii) are present in a ratio by weight of between 1:10 and 10 000:1.
(19) The process for preparing a solid self-microemulsifying system according to item 18, wherein the self-microemulsifying system comprises
   (i) a polyoxyethylene sorbitan fatty acid ester emulsifier,
   (ii) a co-emulsifier selected from glyceryl mono- or di- fatty acid esters of C6-C18 fatty acids; and
   (iii) an oil, selected from C6-C12 fatty acid triglycerides; wherein the polyoxyethylene sorbitan fatty acid ester emulsifier (i) and co-emulsifier (ii) are present in a ratio by weight of between 1:1 and 4:1 and the emulsifier and co-emulsifier, (i) + (ii) and oil (iii) are present in a ratio by weight of between 4:1 and 10 000:1.
(20) The process for preparing a solid self-microemulsifying system according to any one of items 15 to 19, wherein the active pharmaceutical ingredient is fenofibrate.
(21) The process according to items 15 to 17, wherein the active pharmaceutical ingredient is fenofibrate and solid self-microemulsifying system comprises miglyol 812 and polysorbate 80; or polysorbate 20 and isopropyl myristate.
(22) The process according to item 20, wherein miglyol 812 amounts for 1-50 wt. % and polysorbate 80 for 10-99 wt. % of the self-microemulsifying system.
(23) The process according to item 20, wherein polysorbate 20 amounts for 10-99 wt. % and isopropyl myristate for 1-50 wt. % of the self-microemulsifying system.
(24) The process according to any one of previous items, further comprising preparing a medicine.
(25) The process according to item 24 comprising filling the solid self-microemulsifying system into a capsule, compressing it into a tablet or molding it into suppository, optionally together with other pharmaceutically acceptable excipients.
(26) A solid self-microemulsifying system obtainable by the processes defined in any one of the items 1 to 25.
(27) A solid self-microemulsifying system comprising microcrystalline cellulose in a core and viscosity enhancer and seif-microemulsifying system in a layer deposited on the core.
(28) The solid self-microemulsifying system according to item 27, wherein all components form matrix type granule.
(29) The solid self-microemulsifying system according to item 27 or 28, wherein the viscosity enhancer is selected from the group consisting of hydroxypropylmethyl cellulose, hydroxypropyl cellulose, hydroxyethyl cellulose, methylcellulose, carboxymethylcellulose, polyvinyl alcohols, polyvinylpirrolidones, copolyvidones, natural gums such as acacia, tragacanth, xanthan, carageenans, guar, pectin, sugars such as for example glucose, fructose, alginates, carbomers, gelatin, polyethylene glycols and colloidal silicon dioxide,preferably is hydroxypropylmethyl cellulose.
(30) The solid self-microemulsifying system according to any one of items 27 to 29, being further coated with a wet granulation binder, preferably with a hydroxypropylmethyl cellulose, hydroxypropyl cellulose, hydroxyethyl cellulose, polyvinylpirrolidone, copolyvidone, methylcellulose, carboxymethylcellulose, gelatin, natural gums, such as acacia, tragacanth, guar, pectin, or mixture thereof, more preferably with hydroxypropylmethyl cellulose, hydroxypropyl cellulose, hydroxyethyl cellulose, methylcellulose, ethylcellulose, carboxymethylcellulose, polyvinylpirrolidone, copolyvidone, or mixture thereof, particularly with hydroxypropylmethyl cellulose.
(31) A solid self-microemulsifying system comprising a solid self-microemulsifying system in a core and a layer comprising a wet granulation binder deposited on the core, preferably the granulation binder is hydroxypropylmethyl cellulose, hydroxypropyl cellulose, hydroxyethyl cellulose, polyvinylpirrolidone, copolyvidone, methylcellulose, carboxymethylcellulose, gelatin, natural gums, such as acacia, tragacanth, guar, pectin, or mixture thereof, more preferably is hydroxypropylmethyl cellulose, hydroxypropyl cellulose, hydroxyethyl cellulose, methylcellulose, ethylcellulose, carboxymethylcellulose, polyvinylpirrolidone, copolyvidone, or mixture thereof, particularly is hydroxypropylmethyl cellulose.
(32) The solid self-microemulsifying system according to item 31, wherein the solid self-microemulsifying system in the core is as defined in any one of items 27 to 30.
(33) The solid self-microemulsifying system according to any one of items 26 to 32 comprising fenofibrate.
(34) The solid self-microemulsifying system according to any one of items 26 to 33, wherein the solid self-microemulsifying system comprises miglyol 812 and polysorbate 80; or polysorbate 20 and isopropyl myristate.
(35) The solid self-microemulsifying system according to any one of items 26 to 34, wherein miglyol 812 amounts for 1 - 50 wt. % and polysorbate 80 for 10 - 99 wt. % of the self-microemulsifying system.
(36) The solid self-microemulsifying system according to any one of items 26 to 35, wherein polysorbate 20 amounts for 10 - 99 wt. % and isopropyl myristate amounts for 1 - 50 wt. % of the self-microemulsifying system.
(37) Use of a solid self-microemulsifying system according to any one of items 26 to 36 for preparing a medicine.
(38) Use of a microcrystalline cellulose and a viscosity enhancer in admixture with self-microemulsifying system.
(39) Use according to item 38, wherein the viscosity enhancer is selected from the group consisting of hydroxypropylmethyl cellulose, hydroxypropyl cellulose, hydroxyethyl cellulose, carboxymethylcellulose, polyvinyl alcohols, polyvinylpirrolidones, natural gums such as acacia, tragacanth, xanthan, carageenans, sugars such as glucose, fructose, alginates, carbomers, gelatin, polyethylene glycols, colloidal silicon dioxide, preferably is hydroxypropylmethyl cellulose.
(40) Use of a microcrystalline cellulose in a spray-dried self-microemulsifying system for increasing processability of said self-microemulsifying system.
(41) A self-microemulsifying system comprising fenofibrate and miglyol 812 and polysorbate 80; or polysorbate 20 and isopropyl myristate.
(42) The self-microemulsifying system according to item 41, wherein miglyol 812 amounts for 1 - 50 wt. % and polysorbate 80 for 10 - 99 wt. % of the self-microemulsifying system; or polysorbate 20 amounts for 10 - 99 wt. % and isopropyl myristate amounts for 1 - 50 wt. % of the self-microemulsifying system.
(43) A medicine comprising a solid self-microemulsifying system according to any one of items 26 to 36.
(44) Use of a microcrystalline cellulose and a viscosity enhancer for preparing a spray-dried self-microemulsifying system.

### Brief description of the drawings

- Fig. 1: Phase diagram for isopropyl myristate (IPM), fenofibrate (NDS9), polysorbate 20 (Tween 20) and water. Dots in the phase diagram indicate the formation of a microemulsion.
- Fig. 2: Phase diagram for isopropyl myristate (IPM), fenofibrate (NDS9), polysorbate 20 (Tween 20) and water. Dots in the phase diagram indicate the formation of a microemulsion.
- Fig. 3: Phase diagram for fenofibrate (NDS9), polysorbate 20 (Tween 20) and water. Dots in the phase diagram indicate the formation of a microemulsion.
- Fig. 4: Phase diagram for fenofibrate (NDS9), polysorbate 20 (Tween 20) lecitin and water. Dots in the phase diagram indicate the formation of a microemulsion.

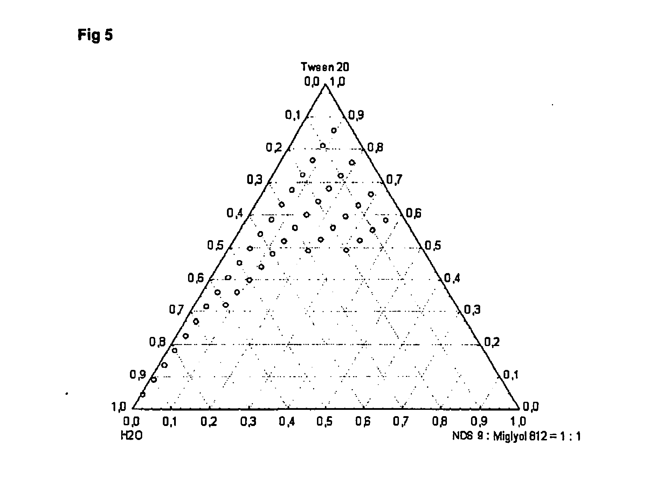

- Fig. 5: Phase diagram for Miglyol, fenofibrate (NDS9), polysorbate 20 (Tween 20) and water. Dots in the phase diagram indicate the formation of a microemulsion.

### Detailed description of the invention

Microemulsions (ME) are clear, stable, isotropic liquid mixture of lipophilic phase, aqueous phase and surfactant, frequently in combination with other excipients. The aqueous phase may contain salt(s) and/or other ingredients, and the lipophilic phase may actually be a complex mixture of different hydrocarbons, lipids or other lipophilic materials. In contrast to coarse (classical) emulsions, microemulsions are formed upon simple mixing of the components and do not require the high energy conditions generally used in the formation of coarse emulsions. Microemulsions have many advantages over coarse emulsion, including thermodynamic stability, greater drug solubilization capacity and permeability enhancement. Because their droplet size is smaller than that of coarse emulsions, they have a greater specific surface area and drug dissolution from microemulsions is consequently faster. In addition, the intra- and inter-patient variability of pharmacokinetic parameters is reduced when a drug is administered in the form of a microemulsion.

Self-microemulsifying systems (SMES) are liquid systems consisting of a lipophilic phase (oil) with one or more surface active substance and/or other excipients, which spontaneously form microemulsions upon the contact with the aqueous media. SMES can be prepared by any known process in the art, e.g. mixing, from any suitable combination of oil and surface active substance(s). Oil and surfactant(s) used can be for example those described in WO 01/01960 A1 document. Compared to microemulsions, they do not contain water and thus exhibit better physical and microbiological stability. They share all the advantages of microemulsions over coarse emulsion. Incorporation of drugs in self microemulsifying systems thus offers several advantages for their delivery, the main one being faster drug dissolution and absorption. Formulation of SMES into solid dosage forms can be particularly difficult.

Surprisingly, it was found that a process for preparing a solid self-microemulsifying system comprising dispersing microcrystalline cellulose in a solvent, wherein the solvent further contains a viscosity enhancer, mixing the solvent with a self-microemulsifying system and spray drying the obtained mixture remarkably enhances yield of the process. The yield is calculated as dry material obtained by a process divided by dry material entering the process. Using microcrystalline cellulose in this first process proved to change properties of the solid SMES obtained in terms of better flowability characteristics and reduced stickiness, which lead to higher percentage of a recovered material. Pinpointing microcrystalline cellulose as a suitable solid support excipient for a spray drying process was completely unexpected since the procedures to convert SMES into solid form using spray drying process described in the aforementioned application only describe soluble excipients or excipients providing molecular or highly colloidal dispersions when placed into water. On the other hand, microcrystalline cellulose used according to the present invention is insoluble. By insoluble it is meant herein that substance is insoluble in water (solubility below 1 µmol/mL) at 25 °C and 1 bar, but may interact with water - e.g. swell. Microcrystalline cellulose is also different from excipients that readily mix with water to form colloidal dispersions (e.g. colloidal silicon dioxide). Such dispersions contain highly colloidally dispersed or even molecularly dispersed excipients and may be thus considered as solutions and as such not considered as insoluble and are not in the context of the present invention. Further, selecting insoluble microcrystalline cellulose was unpredictable since insoluble excipients are not routinely used as solid support in spray drying processes due to processability problems, final product homogeneity as well as potential suboptimal performance due to lower specific surface which is available for the interaction/carrier function for an active pharmaceutical ingredient. Generally, a homogeneous molecular level dispersion of the active pharmaceutical ingredient is desired when using soluble solid supports in spray drying. Similarly, when colloidal solid support is used, large specific surface area is desired for active pharmaceutical ingredient adsorption in order to achieve molecular level drug distribution and meet homogeneity requirement Insoluble solid supports are therefore not commonly used, since they are suboptimal in comparison to soluble solid supports. When converting liquid self-microemulsifying systems into solid form via spray drying the above mentioned general requirements still stand and are even increased in regard of specific surface area, since the amount of SMES to be included in the system is far greater than an amount of pure active ingredient. Consequently insoluble solid supports are theoretically even less suitable and were therefore practically not considered as candidates for solid supports. Furthermore, not even all insoluble solid support excipients worked. Calcium hydrogen phosphate, well known insoluble filler used in the pharmaceutical industry, was shown to be unsuitable for the purpose, but on the other hand, microcrystalline cellulose (MCC), showed unexpected increase in the yield of the process compared to previously described processes.

MCC used according to the present invention has particle size of at least 1 µm, preferably at least 2 µm. Commercially available MCC has a particle size of at least 10 µm, thus preferably particle size of MCC used is from 10 µm to 500 µm, more preferably 20 µm to 200 µm, particularly 50 µm to 100 µm. Specifically it is of importance that particles of MCC, when dispersed in a solvent for spray drying, do not resemble molecularly or highly colloidally dispersed solid support, like for example colloidal silicon dioxide in water. When used for spray drying MCC in a solvent represents 20 - 90 wt. %, preferably 30 - 80 wt. %, more preferably 30 to 60 wt. %, particularly 40 to 60 wt. % based on the final solid self-microemulsifying system. Particle size can be determined by any method known to the skilled person, e.g. by laser diffraction (e.g. Malvern Mastersizer), or simply by sieving. In the event Malvem Mastersizer is used refractive index of substance is set to 1.520, absorption of the substance is set to 0.1, refractive index of dispersant is set to 1.330, sensitivity is set to normal and all other parameters (e.g. obscurity) are within the range required for a correct measurement. Particle size is calculated by the apparatus according to the simplified Mie theory.

In EP1961412A1 only addition of soluble or colloidal suspension forming excipients are described due to processability problems associated with spray drying of insoluble excipients, which form classical suspensions. Said processability problems include sedimentation of insoluble excipients, non-homogeneous product and clogging of tubing and nozzles. In order to properly utilize MCC, said hindrances were overcome by increasing the viscosity of the suspension by adding a viscosity enhancer. Already use of MCC alone, but particularly use of a combination of MCC and the viscosity enhancer for spray drying tremendously facilitated processability of the suspension and increased yields of the process, partly due to better flowability of the mass. Processability is understood herein as better yield, flowability, ability to formulate self-microemulsifying system into solid state composition, and/or facilitated further processing of solid form SMES into dosage forms such as granules, tablets, or capsules.

The term "viscosity enhancer' as used herein is any pharmaceutically acceptable excipient, preferably polymer, that increases the viscosity of a solvent when added thereto or by any other means prevents the sedimentation of insoluble particles in the solvent. The main interest of the addition of viscosity enhancer is to retard or prevent the sedimentation of insoluble particles in order to allow the processability of the suspension. Particularly suitable is viscosity enhancer selected from the group consisting of hydroxypropylmethyl cellulose, hydroxypropyl cellulose, hydroxyethyl cellulose, carboxymethylcellulose, polyvinyl alcohols, polyvinylpirrolidones, natural gums such as acacia, tragacanth, xanthan, carageenans, sugars such as for example glucose, fructose, alginates, carbomers, gelatin, polyethylene glycols and colloidal silicon dioxide, or mixture thereof, preferably is hydroxypropylmethyl cellulose. Generally, viscosity enhancer can be applied in amount of 0.5 - 20 wt. % of the solvent used for spray drying, but in principle it can be employed in any amount achieving sufficient stabilization of the MCC suspension, either before SMES is mixed with the solvent or after it. The skilled person will easily determine without any undue effort the proper amount of any specific viscosity enhancer to prevent MCC from settling. In a specific embodiment, only one viscosity enhancer is present in the composition.

In alternative, the present invention provides a second process for preparing a solid self-microemulsifying system that also significantly increases flowability of the obtained material and is particularly suitable as intermittent process applied prior to formulating or moulding a pharmaceutical composition, optionally with additional pharmaceutically acceptable excipients, into final dosage form. The process comprises mixing a solid support and self-microemulsifying system to form a primary mixture, which can be optionally achieved in a presence of a granulating or spray drying liquid, and further granulating said primary mixture with a solution or suspension of a granulation binder. Primary mixture can be understood as a first granulate, which gets granulated a second time. This procedure unexpectedly improved flowability in a simple and straightforward way and in addition allowed final dosage form to be formulated by using only limited amount of excipients. Said granulation binder, i.e. wet granulation binder, used for granulating can be any known pharmaceutically acceptable excipient used for granulation or coating, like for example hydroxypropylmethyl cellulose, hydroxypropyl cellulose, hydroxyethyl cellulose, polyvinylpirrolidone, copolyvidone, methylcellulose, carboxymethylcellulose, gelatin, natural gums, such as acacia, tragacanth, guar, pectin, or mixture thereof, preferably is hydroxypropylmethyl cellulose, hydroxypropyl cellulose, hydroxyethyl cellulose, methylcellulose, ethylcellulose, carboxymethylcellulose, polyvinylpirrolidone, copolyvidone, or mixture thereof, particularly is hydroxypropylmethyl cellulose. This alternative process can be easily applied after the first process is already carried out, i.e. to coat solid SMES comprising MCC obtainable by the first aforementioned process.

A "solid support" as used herein refers to any pharmaceutically acceptable excipient that is soluble or insoluble in a self-microemulsifying system or granulating or spray drying liquid. It should allow deposition of SMES either on its surface or within its matrix, either while in a liquid or after being dried. The said pharmaceutical acceptable excipient is in solid state at the room conditions (25°C and 1 bar), serving as a filler and thus providing the final mixture with favourable physical characteristics for further processing. For example, the solid support can be lactose, microcrystalline cellulose, dibasic and tribasic calcium phosphate, silicon dioxide, kaolin, bentonite, hetorite, magnesium trisilicate, aluminium hydroxide, magnesium hydroxide, magnesium oxide, mannitol, talc, magnesium stearate, sodium chloride, potassium chloride, citric acid, starches and its derivatives, powdered cellulose and other cellulose derivatives, sorbitol, sucrose, sucrose-based materials, calcium sulfate, sorbitol, xylitol, dicalcium hydrogen phosphate, or mixture thereof.

Solvent used in first and second process, either as the solvent, granulating or spray drying liquid, or to form the solution or suspension can be selected from the group consisting of aqueous and organic solvents or a mixture thereof. Normally water, ethanol, acetone, isopropanol, or a mixture thereof will be used, particularly water.

Any pharmaceutically active ingredient can be incorporated into the SMES or added thereto. Pharmaceutically active ingredient can be a small molecule drug (i.e. with molecular mass up to 1500 g/mol, preferably up to 1000 g/mol), nutritional supplement (e.g. a vitamin), peptide or protein, or a combination thereof. The invention is particularly useful in connection to oral administration of poorly soluble drugs, i.e. those that display low dissolution in physiological fluids, which may results in poor bioavailability. By poorly soluble we mean the ingredient has a solubility of less than 0.1 g/ml of water at 37°C, as defined for practically insoluble drugs in USP29. The solubility of the active pharmaceutical ingredient (or of MCC) can be determined by any technique known to those skilled in the art, like for example by way of placing an excess of the active pharmaceutical ingredient (or MCC) in a defined amount of water, dissolving it to the extent where equilibrium is reached, and measuring the amount dissolved by a suitable analytical technique. Convenient analytical techniques can be measuring UV absorbance or HPLC. Active pharmaceutical ingredient is preferably selected from the group consisting of acetohexamide, albendazole, alendronate, amiodarone, aprepitant, atovaquone, bendroflumetiazid, benzthiazide, betamethasone, cyclosporine, danazol, diazepam, dutasteride, ezetimibe, felodipine, phenylbutazone, flufenaminic acid, fenofibrate, furosemide, glibenklamide, glutethimide, griseofulvin, hydroflumethiazide, hydrochlortiazide, hydrocortisone, ibuprofen, indomethacin, itraconazole, carbamazepine, ketoconazole, ketoprofen, clofibrate, chloramphenicol, chlorothiazide, clorpropamide, mebendazole, mefenaminic acid, megestrol, naproxen, nimodipin, nitrazepam, nitrendipine, nitrofurantoin, oxazepam, pioglitazone, prazosin, prednisolone, reserpine, simvastatine, sirolimus, spironolactone, telmisartan and troglitazone, or mixture thereof.

In a specific embodiment of the present invention, which can stand alone or in combination with any one of the aforementioned alternatives, fenofibrate is used as an active pharmaceutical ingredient. It is well known for its poor solubility. It has been reformulated from microparticle formulation throughout nanoparticle formulation into solid dispersion (molecularly dispersed active ingredient) all with the intent of increasing its solubility and consequently its bloavailability. Due to increased bioavailability the active ingredient dose has been reduced from formulation to formulation. Decrease in dose usually results in decreased incidence of adverse reactions, as well as decreased expenses due to active ingredient dose reduction. Increase in dissolution is therefore required and SMES offer an excellent opportunity since the drug is molecularly dispersed and high concentration of surface active ingredients are present which can further boost the dissolution and absorption. On the other side, the therapeutic dose of fenofibrate is relatively high (above 100 mg), which requires SMES systems with great solubilization capacity for fenofibrate in order to prepare a suitably sized final dosage form. For illustration: if the solubility of fenofibrate is 10% in SMES and the dose is 100 mg, 1000 mg of SMES and fenofibrate needs to be formulated into a solid formulation, an amount which per se is on the border of suitably sized oral dosage form. With the help of improved processed of the present invention, solid compositions of SMES comprising fenofibrate in high load and at the same time with only acceptable amount of excipients can be easily prepared. Particularly advantageous is formulation comprising fenofibrate and SMES comprising miglyol 812 and polysorbate 80; or polysorbate 20 and isopropyl myristate. Miglyol 812 can amount for 1-50 wt. % and polysorbate 80 for 10-99 wt. % of the self-microemulsifying system. In alternative, polysorbate 20 can amount for 10-99 wt. % and isopropyl myristate for 1-50 wt. % of the self-microemulsifying system. Said fenofibrate systems can be easily used in the processes of the present invention.

In another specific embodiment SMES used in a process or in a composition according to the present invention comprises
(i) a polyoxyethylene sorbitan fatty acid ester emulsifier;
(ii) a co-emulsifier selected from glyceryl mono- or dl- fatty acid esters of C6-C18 fatty acids; and
(iii) an oil, selected from C6-C12 fatty acid triglycerides;
wherein the polyoxyethylene sorbitan fatty acid ester emulsifier (i) and co-emulsifier (ii) are present in a ratio by weight of between 1:1 and 8:1, preferably between 1:1 and 4:1, and the emulsifier and co-emulsifier, (i) + (ii) and oil (iii) are present in a ratio by weight of between 1:10 and 10 000:1, preferably between 4:1 and 10 000:1

Any embodiment of the present invention can be further evolved to a stage where a medicine is prepared. Steps to arrive at the medicine will depend on an active pharmaceutical ingredient used and animal or human to be treated. Further they will depend on physiological status or severity of pathological complication, dose of the active pharmaceutical ingredient used, route of administration chosen etc. The skilled person will know how to apply general knowledge to arrive at the proper medicine. For example, standard techniques of pharmaceutical technology can be employed, e.g. filling the obtained solid self-microemulsifying system into a capsule, compressing it into a tablet or molding it into suppository, optionally together with other pharmaceutically acceptable excipient. Said excipients can be for example filler, binder, colorant, pH modifier, desintegrant, cosolvent, solvent, pigment, diluent, glidant, surfactant.

First process of the present invention produces a solid self-microemulsifying system. When a single MCC particle is analysed, the solid self-microemulsifying system comprises microcrystalline cellulose in a core and viscosity enhancer and self-microemulsifying system in a layer deposited on the core. Granules of it comprise components forming matrix. The product has good flowability characteristics and low stickiness. Alternatively, the product can be further coated with a wet granulation binder, which can be done by second process. In any event, a solid self-microemulsifying system obtainable by the second process comprises a solid self-microemulsifying system in a core and a layer comprising a wet granulation binder deposited on the core. Wet granulation binder can be for example hydroxypropylmethyl cellulose, hydroxypropyl cellulose, hydroxyethyl cellulose, polyvinylpirrolidone, copolyvidone, methylcellulose, carboxymethylcellulose, gelatin, natural gums, such as acacia, tragacanth, guar, pectin, or mixture thereof, preferably is hydroxypropylmethyl cellulose, hydroxypropyl cellulose, hydroxyethyl cellulose, methylcellulose, ethylcellulose, carboxymethylcellulose, polyvinylpirrolidone, copolyvidone, or mixture thereof, particularly is hydroxypropylmethyl cellulose. Highly advantageous is to have a product obtained by the first process further coated by hydroxypropylmethyl cellulose, hydroxypropyl cellulose, hydroxyethyl cellulose, methylcellulose, ethylceflulose, carboxymethylcellulose, polyvinylpirrolidones, copolyvidones, preferably hydroxypropylmethyl cellulose. Said solid self-microemulsifying system can comprise any aforementioned active pharmaceutical ingredient and can be conveniently used for preparing a medicine.

The following examples are merely illustrative of the present invention and they shall not be considered as limiting the scope of the invention in any way, as these examples and other equivalents thereof will become apparent to those versed in the art in the light of the present disclosure, and the accompanying claims. For illustrative purposes, the principle, advantages and preferred embodiments of the present invention are shown by the use of fenofibrate and simvastatin as exemplified active pharmaceutical ingredients, while it is to be appreciated that the examples and modifications thereof are likewise applicable to other active pharmaceutical ingredient.

### EXAMPLE 1:

Preparation of SMES:

| ingredients | SMES |
|---|---|
| Miglyol 812 | 20.00 % |
| Polysorbate 80 | 60.00 % |
| Imwitor 308 | 20.00 % |

Procedure: Imwitor 308 (coemulsifier) was heated in water bath at 40 °C, then Polysorbate 80 (emulsfier) was added. Both ingredients were mixed thoroughly. Mixture was allowed to cool and then Miglyol 812 (oil phase) was added and stirred.

Spray dried microemulsions SMES were prepared by dissolving or dispersing solid support in water. This dispersion was mixed together with SMES. The obtained dispersion was spray dried in a spray drying system.

Polyvinylpyrrolidone and colloidal silicone dioxide (samples 1a and 1b, respectively) were chosen as suitable prior art solid supports. In addition, colloidal silicone dioxide and calcium hydrogen phosphate (Sample 2a and 2b, respectively) were tested as solid supports, both supplemented by addition of hydroxypropyl methylcellulose, which was allowed to dissolve before spray drying.

All samples were stirred during spray drying. Spray drying for all samples was performed at constant parameters.

Compositions and yields of spray dried SMES preparations

| **Ingredient (g)** | **Sample 1a** | **Sample 1b** | **Sample 2a** | **Sample 2b** |
|---|---|---|---|---|
| SMES1 | 2,00 | 1 | 1 | 2,00 |
| Water | 18,00 | 23,3 | 23,3 | 18,00 |
| HPMC | - | - | 0,759 | 0,66 |
| MCC Grade 1 | - | - | - | - |
| MCC Grade 2 | - | - | - | - |
| CaHPO₄ | - | - | - | 2,00 |
| Polyvinylpyrrolidone | 2,00 | - | - | - |
| Colloidal silicon dioxide | - | 1 | 1 | - |
| **Yield (%)** | **15,3** | **46,6** | **46,5** | **45,6** |

Sample with polyvinylpyrrolidone exhibited extremely poor yield and sample with colloidal silicon dioxide exhibited mediocre yield. The addition of hydroxypropylmethyl cellulose (HPMC) to the colloidal silicone dioxide dispersion did not have an influence on the total yield as seen from the comparison of sample 1b and 2a. Thus, HPMC alone does not improve yield on its own. In addition, insoluble solid support, such as calcium hydrogen phosphate in combination with HPMC also does not improve yield.

### EXAMPLE 2:

SMES was prepared according to the example 1. Spray dried microemulsions were also prepared according to example 1.

Microcrystalline cellulose of two different grades was tested as solid support. MCC grade 1 had particles of 50 µm size and MCC of grade 2 had them of 100 µm size. Hydroxypropyl methylcellulose was added to both samples and allowed to dissolve before spray drying. All samples were stirred during spray drying. Spray drying for all samples was performed at constant parameters as in the example 1.

Compositions and yields of spray dried SMES preparations

| **Ingredient (g)** | **Sample 3a** | **Sample 3b** |
|---|---|---|
| SMES1 | 2,00 | 2,00 |
| Water | 18,00 | 18,00 |
| HPMC | 0,66 | 0,66 |
| MCC Grade 1 | 2,00 | - |
| MCC Grade 2 | - | 2,00 |
| CaHPO₄ | - | |
| Polyvinylpyrrolidone | - | - |
| Colloidal silicon dioxide | - | - |
| **Yield (%)** | **84,4** | **85,4** |

Both samples exhibited significantly higher yield compared to previous examples. The yield was not affected by the MCC grade.

From example 1 and 2 it can be deduced that the addition of hydroxypropyl methylcellulose by itself does not guarante to increase the process yield, since the addition of hydroxypropyl methylcellulose to calcium hydrogen phosphate or colloidal dispersions of fumed silica did not improve the processability of the suspension. On the other hand, a combination of MCC and HPMC improved yield significantly. This further confirmed that the formulation of microcrystalline cellulose and hydroxypropyl methylcellulose indeed presents some unexpected benefits.

### EXAMPLE 3:

### Granulate composition:

| **ingredient** | **Sample 4a** |
|---|---|
| MCC Grade 1 | 12,5 g |
| SMES1 | 12,5 g |

### Manufacturing procedure:

SMES1 was prepared according to the example 1 in EP1961412 A1. SMES1 was mixed with MCC to form a mixture with SMES1 adsorbed or absorbed onto MCC. Granulate was assessed in terms of flowability. Final granulate was mixed with 1% colloidal silica, a standard method for increasing flowability, and flowability was measured again.

### Results:

| | **Sample 4a** | **Sample 4a + colloidal silica** |
|---|---|---|
| Flow time (s/100g) | Not measurable | Not measurable |
| Angle of repose | Not measurable | Not measurable |

The mixing of the components resulted in sticky and oily mass without any flowability. The addition of colloidal silica did not improve the flowability.

### EXAMPLE 4:

### Granulate composition:

| **Ingredient** | **Sample 5a** | **Sample 5b** |
|---|---|---|
| MCC Grade 1 | 12,5 g | 12,5 g |
| SMES1 | 12,5 g | 12,5 g |
| HPMC | 0,2 g | 0,4 g |

### Manufacturing procedure:

SMES1 was prepared according to the example 1 in EP1961412 A1. SMES1 was mixed with MCC to form the primary mixture. Primary mixture was further granulated with 4% aqueous solution of HPMC to obtain final granulates, which were dried on a try dryer. Granulates were assessed in terms of flowability. Final granulates were mixed with 1% colloidal silica, a standard method for increasing flowability, and flowability was measured again.

### Results:

| | **Sample 5a** | **Sample 5b** | **Sample 5a + colloidal silica** | **Sample 5b + colloidal silica** |
|---|---|---|---|---|
| Flow time (s/100g) | 300 | 226,2 | 118,8 | 70,3 |
| Angle of repose | 29,9 | 35,5 | 26,6 | 35,1 |

The flowability of the samples was significantly improved compared to the primary mixture as a consequence of the granulation. The addition of colloidal silica further improved the flowability. Compared to the example 4 the addition of colloidal silica improved the flowability.

### EXAMPLE 5:

### Granulate composition:

| **Ingredient** | **Sample 6a** | **Sample 6b** |
|---|---|---|
| Miglyol 812 | 13,33 | - |
| Polysorbate 80 | 133,33 | - |
| isopropyl myristate | - | 20,00 |
| Polysorbate 20 | - | 150,00 |
| Fenofibrat | 120,00 | 120,00 |
| MCC | 266,67 | 274,00 |
| HPMC | 17,73 | 16,00 |
| Deminaralized water | 160,00 | 160,00 |

### Manufacturing Procedure:

Surfactants (Polysorbate 80 or 20) were mixed with lipophilic phase (Miglyol or isopropyl myristate) and heated to 80 °C. Fenofibrate was added, allowed to melt and clear SMES was formed. Liquid heated SMES was poured onto MCC and mixed to obtain the primary mixture. This mixture was further granulated with aqueous HPMC solution to obtain final granulates. Granulates were dried in a vacuum dryer at 40 °C to obtain dry granulate. Granulates were compressed into tablets on an eccentric tablet press.

### EXAMPLE 6:

SMES1 and spray dried microemulsions were prepared according to the example 1. Simvastatin was dissolved in SMES1 prior to mixing with water. Microcrystalline cellulose and hydroxypropyl methylcellulose were used as described in example 2. Different concentrations of both excipients and SMES1 in the final solution for spray drying were tested. All samples were stirred during spray drying. Spray drying for all samples was performed at constant parameters as in the example 1.

Compositions and yields of spray dried SMES preparations

| **Ingredient (g)** | **SMES1** | **Water** | **HPMC** | **MCC Grade 2** | **Yield (%)** |
|---|---|---|---|---|---|
| **Sample 6a** | 11,8 | 69 | 2,54 | 5,7 | 65,5% |
| **Sample 6b** | 14,4 | 60 | 2,00 | 3,6 | 84,6% |
| **Sample 6c** | 11,8 | 69 | 2,53 | 5,6 | 86,5% |
| **Sample 6d** | 10,1 | 60 | 2,66 | 7,2 | 94,0% |
| **Sample 6e** | 9,6 | 60 | 3,20 | 7,2 | 94,0% |
| **Sample 6f** | 8,18 | 71 | 3,64 | 8,2 | 88,5% |
| **Sample 6g** | 14,4 | 60 | 1,60 | 4,0 | 62,0% |
| **Sample 6h** | 10,3 | 94 | 4,57 | 5,1 | 64,5% |
| **Sample 6i** | 11,8 | 69 | 2,53 | 5,6 | 82,4% |
| **Sample 6j** | 10,6 | 98 | 2,35 | 7,0 | 81,0% |
| **Sample 6k** | 8,78 | 78 | 3,90 | 7,3 | 85.5% |
| **Sample 6l** | 13,0 | 88 | 2,16 | 4,9 | 67.1% |
| **Sample 6m** | 13,6 | 60 | 2,80 | 3,6 | 76,9% |
| **Sample 6n** | 8,7 | 77 | 2,58 | 8,7 | 63,9% |
| **Sample 6o** | 11,6 | 109 | 2,58 | 5,8 | 78,1% |
| **Sample 6p** | 14,0 | 73 | 1,86 | 4,2 | 68,1% |
| **Sample 6r** | 11,8 | 69 | 2,53 | 5,6 | 85,4% |
| **Sample 6s** | 12,3 | 60 | 1,60 | 6,1 | 87,0% |
| **Sample 6t** | 13,2 | 60 | 3,20 | 3,6 | 87,0% |
| **Sample 6u** | 12,0 | 60 | 3,20 | 4,8 | 76,5% |
| **Sample 6v** | 11,2 | 60 | 1,60 | 7,2 | 73,0% |
| **Sample 6z** | 11,5 | 80 | 4,00 | 4,5 | 97,0% |
| **Sample 6x** | 10,7 | 99 | 3,97 | 5,4 | 71,9% |
| **Sample 6y** | 9,0 | 80 | 2,00 | 9,0 | 89,0% |

All samples exhibited significantly higher yield compared samples 1a and 1b, and samples 2a and 2b.

### EXAMPLE 7:

### Granulate composition:

| **Ingredient** | **Sample 7a** | **Sample 7b** |
|---|---|---|
| MCC Grade 1 | 32 g | 32 g |
| SMES1 | 64 g | 64 g |
| HPC | 3,4 g | 3,4g |
| | | |

### Manufacturing procedure:

SMES1 was prepared according to the example 1. SMES1 was mixed with MCC to form the primary mixture. Primary mixture was further granulated with 10% aqueous (Sample 7a) or ethanol (Sample 7a) solution of HPC to obtain final granulates, which were dried on a try dryer under vacuum. Granulates were assessed in terms of flowability.

### Results:

| | **Primary mixture** | **Sample 7a** | **Sample 7b** |
|---|---|---|---|
| Flow time (s/100g) | Not measurable | 118 | 81 |
| Angle of repose | Not measurable | 40,2 | 37,7 |

The flowability of the primary mixture (MCC and SMES1) could not be measured due to stickiness and virtually no flowability properties of the mixture. The flowability of the samples was significantly improved compared to the primary mixture as a consequence of the granulation.

This example shows that any viscosity enhancer would work, not just HPMC. In addition, it clearly shows that different solvents (Sample 7a) can be used In the process.

### EXAMPLE 8:

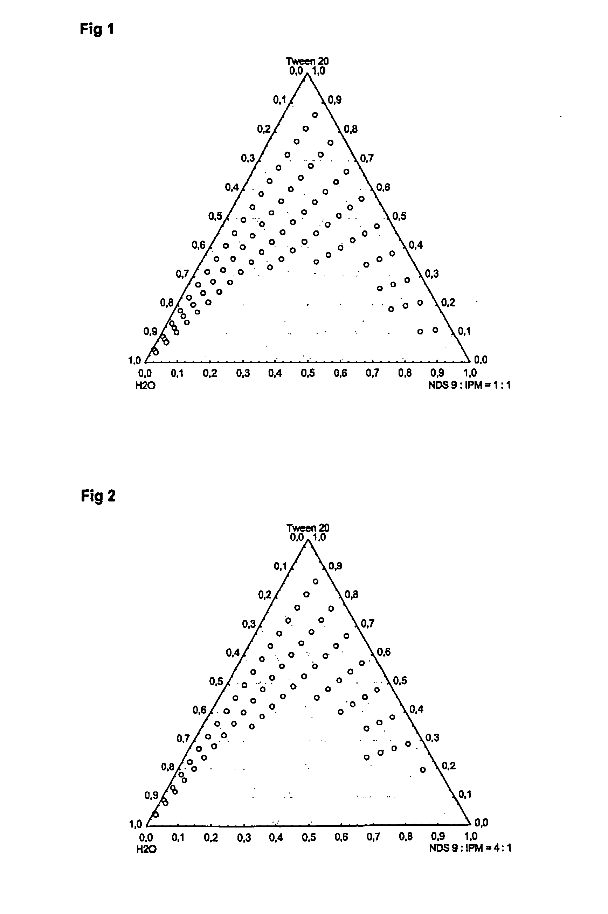

Surfactant polysorbate 20 was mixed with lipophilic phase isopropyl myristate and heated to 80 °C. Fenofibrate was added, allowed to melt and clear SMES was formed. Different mixtures with varying ratios of surfactant to lipophilic phase and fenofibrate to lipophilic phase were prepared and water was added while the temperature was maintained at 80°C. The formation of microemulsion was optically determined. Dots in the phase diagrams in Figures 1 and 2 represent the formation of a microemulsion. The formation of microemulsion indicates that the mixture of substances before the addition of water is indeed a SMES. Compositions with lower amount of surfactants and higher amount of the active substance are preferred. Higher dilution potential (SMES without water can accept high proportion of water and the microemulsion is still formed) is preferred.

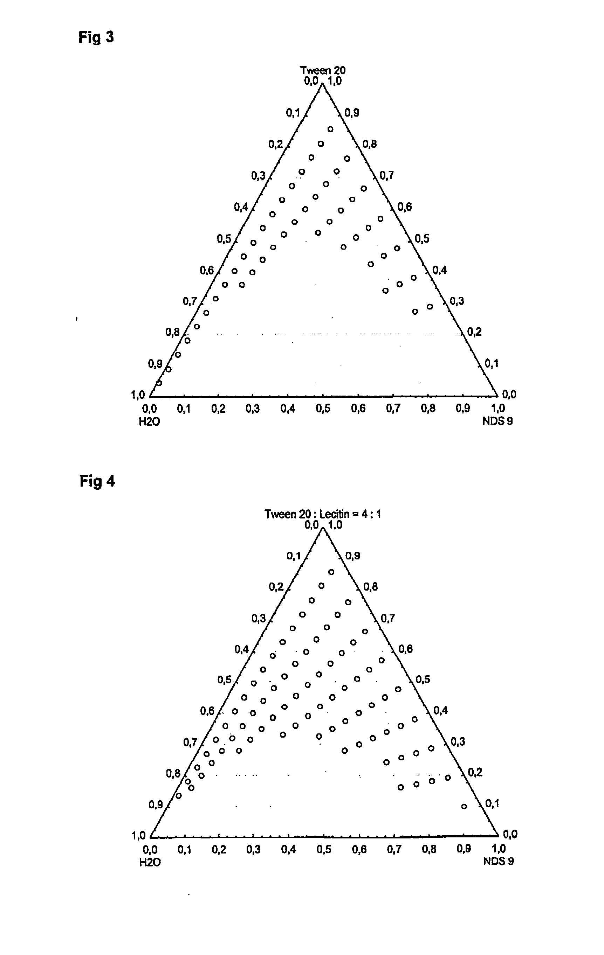

Surfactant polysorbate 20 was heated to 80 °C. Fenofibrate was added, allowed to melt and clear SMES was formed. Different mixtures with varying ratios of fenofibrate to surfactant were prepared and water was added while the temperature was maintained at 80°C. The formation of microemulsion was optically determined. Dots in the phase diagram in Figure 3 represent the formation of a microemulsion.

Surfactant polysorbate 20 was mixed with lecithin and heated to 80 °C. Fenofibrate was added, allowed to melt and clear SMES was formed. Different mixtures with varying ratios of fenofibrate to surfactant were prepared and water was added while the temperature was maintained at 80°C. The formation of microemulsion was optically determined. Dots in the phase diagram in Figure 4 represent the formation of a microemulsion.

Surfactant polysorbate 20 was mixed with lipophilic phase Miglyol and heated to 80 °C. Fenofibrate was added, allowed to melt and clear SMES was formed. Different mixtures with varying ratios of surfactant to lipophilic phase and fenofibrate to lipophilic phase were prepared and water was added while the temperature was maintained at 80°C. The formation of microemulsion was optically determined. Dots in the phase diagrams in Figure 1 and 2 represent the formation of a microemulsion.

### EXAMPLE 9:

SMES2 is prepared according to the example 8 and comprises 70% polysorbate 20 and 30% isopropyl myristate. Spray dried microemulsions are prepared according to the example 1. Microcrystalline cellulose of two different grades is tested as solid support. Hydroxypropyl methylcellulose is added to both samples and allowed to dissolve before spray drying. All samples are stirred during spray drying. Spray drying for all samples is performed at constant parameters as in the example 1.

Compositions and yields of spray dried SMES preparations

| **Ingredient (g)** | **Sample 3a** | **Sample 3b** |
|---|---|---|
| SMES2 | 2,00 | 2,00 |
| Water | 18,00 | 18,00 |
| HPMC | 0,66 | 0,66 |
| MCC Grade 1 | 2,00 | - |
| MCC Grade 2 | - | 2,00 |
| CaHPO₄ | - | |
| Polyvinylpyrrolidone | - | - |
| Colloidal silicon dioxide | - | - |
| **Yield (%)** | **82,4** | **81,9** |

## Claims

1. A process for preparing a solid self-microemulsifying system comprising:
a) dispersing microcrystalline cellulose in a solvent,
b) adding a viscosity enhancer in the solvent,
c) mixing the solvent with a self-microemulsifying system to obtain a mixture, and
d) spray drying the mixture.

2. The process for preparing a solid self-microemulsifying system according to claim 1, wherein the viscosity enhancer is selected from the group consisting of hydroxypropylmethyl cellulose, hydroxypropyl cellulose, hydroxyethyl cellulose, methylcellulose, carboxymethylcellulose, polyvinyl alcohols, polyvinylpirrolidones, copolyvidones, natural gums, sugars, alginates, carbomers, gelatin, polyethylene glycols and colloidal silicon dioxide, or mixture thereof.

3. The process for preparing a solid self-microemulsifying system according to claim 1 or 2, wherein the viscosity enhancer is hydroxypropylmethyl cellulose.

4. A process for preparing a solid self-microemulsifying system comprising:
e) mixing a solid support and self-microemulsifying system to form a primary mixture, optionally in a presence of a granulating or spray drying liquid, and
f) further granulating said primary mixture with a solution or suspension of a granulation binder.

5. The process for preparing a solid self-microemulsifying system according to claim 4, wherein the granulation binder is selected from the group of hydroxypropylmethyl cellulose, hydroxypropyl cellulose, hydroxyethyl cellulose, polyvinylpirrolidone, copolyvidone, methylcellulose, carboxymethylcellulose, gelatin, acacia, tragacanth, guar, pectin, or mixture thereof.

6. The process for preparing a solid self-microemulsifying system according to claim 4 or 5, wherein the solid support is lactose, microcrystalline cellulose, dibasic and tribasic calcium phosphate, silicon dioxide, kaolin, bentonite, hetorite, magnesium trisilicate, aluminium hydroxide, magnesium hydroxide, magnesium oxide, mannitol, talc, magnesium stearate, sodium chloride, potassium chloride, citric acid, starch, powdered cellulose, sorbitol, sucrose, calcium sulfate, sorbitol, xylitol, dicalcium hydrogen phosphate, or mixture thereof.

7. The process for preparing a solid self-microemulsifying system according to any one of the previous claims, wherein the solvent is selected from the group consisting of aqueous and organic solvents or a mixture thereof.

8. The process for preparing a solid self-microemulsifying system according to claim 7, wherein the solvent is selected from the group consisting of water, ethanol, acetone, isopropanol, or a mixture thereof.

9. The process for preparing a solid self-microemulsifying system according to any one of claims 4 to 8, wherein solid support is microcrystalline cellulose and the process further comprises steps of claim 1.

10. The process for preparing a solid self-microemulsifying system according to any one of previous claims, the process further comprising adding an active pharmaceutical ingredient.

11. The process according to any one of previous claims, further comprising preparing a medicine.

12. A solid self-microemulsifying system obtainable by the processes defined in any one of the previous claims.

13. Use of a solid self-microemulsifying system according to claim 12 for preparing a medicine.

14. Use of microcrystalline cellulose and a viscosity enhancer for preparing a spray-dried self-microemulsifying system.

15. A medicine comprising a solid self-microemulsifying system according to claim 12.
